# EUROPÄISCHE PATENTANMELDUNG

(11) **EP 3 804 702 A1**
(43) Veröffentlichungstag der Anmeldung: **14.04.2021**
(21) Anmeldenummer: 19202458.6
(22) Anmeldetag: 10.10.2019
(51) Int. Cl.: A61K 9/51, A61K 31/405, A61K 31/506, A61P 9/00

(54) **VERFAHREN ZUR HERSTELLUNG EINER PHARMAZEUTISCHEN FORMULIERUNG MIT WIRKSTOFF, POLYMER UND TENSID**

(71) Anmelder: Bayer AG, 51373 Leverkusen (DE)
(72) Erfinder: Die Erfindernennung liegt noch nicht vor
(74) Vertreter: BIP Patents

(57) **Zusammenfassung**

Ein Verfahren zur Herstellung einer pharmazeutischen Formulierung umfasst die Schritte:
A) Suspendieren eines pharmazeutischen Wirkstoffes in einer wässrigen Lösung eines Polymers;
B) Trocknen der nach Schritt A) erhaltenen Mischung;
wobei in Schritt A) der pharmazeutische Wirkstoff in Form von Partikeln vorliegt, deren d₉₀-Wert der Partikelgrößenverteilung ≤ 1 µm beträgt und vor Schritt B) der pharmazeutische Wirkstoff weiterhin mit einem ionischen Tensid kontaktiert wird.

## Beschreibung

Die vorliegende Erfindung betrifft ein Verfahren zur Herstellung einer pharmazeutischen Formulierung, umfassend die Schritte: A) Suspendieren eines pharmazeutischen Wirkstoffes in einer wässrigen Lösung eines Polymers und B) Trocknen der nach Schritt A) erhaltenen Mischung. Die Erfindung betrifft ebenfalls eine pharmazeutische Formulierung, umfassend einen mit einem zumindest teilweise in Wasser löslichen Polymer beschichteten pharmazeutischen Wirkstoff und eine Suspension eines pharmazeutischen Wirkstoffes.

Eine hohe Auflösungsrate eines pharmazeutischen Wirkstoffes resultiert meistens in einer erhöhten Bioverfügbarkeit, zumindest aber in einer verbesserten Kinetik der Bioverfügbarkeit. Dies kann beispielsweise durch die Vergrößerung der spezifischen Oberfläche des Wirkstoffpartikel-Kollektivs realisiert werden. So weisen Wirkstoffnanosuspensionen eine wesentlich höhere Auflösungsrate als eine mikronisierte Suspension auf. Um feste Darreichungsformen produzieren zu können, müssen die Nanosuspensionen getrocknet werden. Dabei kommt es häufig zur irreversiblen Aggregation der Nanopartikel und somit zur verschlechterten Auflösungsrate des Wirkstoffes. Trocknungsprozesse sind häufig Gefriertrocknung und Sprühtrocknung bzw. weitere auf Versprühen der Suspensionen basierende sowie andere auf Kontakttrocknung basierende Verfahren, wie z.B. Walzentrocknung oder Vakuumwalzentrocknung oder Eintrocknen unter Umgebungs- oder reduziertem Druck. Dabei kommen neben den üblichen Additiven zur Stabilisierung der Nanosuspensionen wie Polymere und Tenside auch Gerüstbildner (verschiedene Zucker und Zuckeralkohole) zum Einsatz.

Chaubal et al. berichtet in "Conversion of Nanosuspensions into Dry Powders by Spray Drying: A Case Study", Pharmaceutical Research, Vol. 25, No. 10, Oktober 2008, von redispergierbaren Nanopartikelenthaltenden Pulvern und der Wichtigkeit von geladenen Tensiden in Bezug auf die Stabilität von Partikeln während der Trocknung. Nachteilig an dieser Methode ist, dass die durchschnittliche Partikelgröße der Suspensionen nach der Redispergierung stets 10-20% größer ist als im Vergleich zu den Ursprungssuspensionen vor der Trocknung. Des Weiteren werden zusätzlich zu den Stabilisierungsadditiven (Poloxamer 188 und Natriumdesoxycholat) Gerüstbildner wie Lactose, Saccharose und Mannitol verwendet. Zusätzlich sind die Partikeln wesentlich gröber (99% < 1µm) (auch vor der Trocknung (99% < 0,8 µm).

Khinast et al. berichtet in "Nano-extrusion: a One-Step Process for Manufacturing of Solid Nanoparticle Formulations Directly from the Liquid Phase", AAPS PharmSciTech, Vol. 14, No. 2, Juni 2013, von festen Nanopartikelnenthaltenden Formulierungen, die durch Extrusion hergestellt wurden. Dabei wurden die Partikeln in eine Polymermatrix eingebettet. Es wurden keine Redispergierversuche durchgeführt und dem entsprechend keine positiven Effekte der Additive diskutiert. Des Weiteren wurden keine Wirkstoffe sondern anorganische Nanopartikeln verwendet (TiO₂).

Wang et al. berichtet in "Stability of nanosuspensions in drug delivery", Journal of Controlled Release 172 (2013) 1126-1141, von durch Gefriertrocknung hergestellten redispergierbaren Nanopartikelnenthaltenden Pulvern. Auch hier wurden neben den üblichen Stabilisatoren zusätzliche Gerüstbildner verwendet. SDS nur als Additiv für Mahlung verwendet

Cerdeira et al., "Formulation and drying of miconazole and itraconazole nanosuspensions", International Journal of Pharmaceutics 443 (2013) 209-220, stellt verschiedene Wirkstoffnanosuspensionen mit HPC und SDS her allerdings mit zu geringen Konzentrationen um nach der Trocknung, eine vollständige Redispergierbarkeit der Nanopartikelenthaltenden Pulver zu erzeugen. Zusätzlich werden auch hier sowohl bei der Sprühtrocknung, als auch bei der Gefriertrocknung, Gerüstbildner (Mannitol) verwendet. Auch hier wird durch die konstant geringe SDS-Konzentration nicht der positive Effekt auf die Stabilität der Nanopartikeln während der Trocknung erkannt. NACHTEIL: keine Redispergierbarkeit ohne Gerüstbildner, große Partikel nach der Trocknung (mehrere Mikrometer). SDS nur als Additiv für Mahlung verwendet.

Dolenc et al (2009), "Advantages of celecoxib nanosuspension formulation and transformation into tablets", International Journal of Pharmaceutics 376 (2009) 204-212, stellt Nano-Suspensionen mit PVP und SDS als Additive her und trocknet diese mittels Sprühtrocknung. Nachteilig an dieser Methode ist, dass die Pulver zwar redispergierbar sind, allerdings sind deutliche Abweichungen im x90 (30%) zwischen der Ursprungs- und redispergierten Suspension zu erkennen. Die mgl. Vorteile von ionischen Tensiden für die Stabilität der Nanopartikeln während der Trocknung sind nicht beschrieben. SDS fungiert nur als Additiv für die Bildung stabiler Nanopartikel mittels Fällung. Zusätzlich liegen wesentlich gröbere Partikeln im x90 -Bereich vor (> 1µm).

Beirowski et al (2011) erläutert verschiedene Mechanismen der Gefriertrocknung von Nanosuspensionen wobei er auf die richtige Wahl von Kryoprotektoren (z.B. Zucker, Zuckeralkohole, Polymere) hinweist. Dabei werden mögliche Effekte von ionischen Tensiden bezogen auf die Trocknung nicht beschrieben.

WO 2007/107222 A1 beansprucht redispergierbare Nanopartikeln, die durch mindestens ein oberflächenmodifizierendes Molekül aus der Gruppe der Thiole, Sulfide, Disulfide oder Polysulfide erhältlich sind und als Radikalkettenüberträger fungieren. Ziel ist die Funktionalisierung der Oberfläche für weitere Reaktionen und nicht die Redispergierbarkeit. Es wurden nur anorganische Partikeln verwendet

US 2011/0064812 A1 beansprucht die Herstellungsmethode bei der feste, orale Darreichungsformen (Wirkstoffnanopartikeln enthaltend) mittels Fischgelatine hergestellt werden. Nachteil ist auch hier, dass zusätzliche Gerüstbildner verwendet werden müssen. Ionische Tenside kommen nicht zum Einsatz.

Die vorliegende Erfindung hat sich die Aufgabe gestellt, eine verbesserte Herstellung von redispergierbaren Wirkstoffpartikel enthaltenden Pulvern bereitzustellen. Dabei soll die Partikelgrößenverteilung (PGV) der Ursprungssuspension nach Redispergierung der getrockneten Pulver weitestgehend erhalten bleiben und die Aggregation der Nanopartikel verhindert werden.

Erfindungsgemäß gelöst wird diese Aufgabe durch ein Verfahren gemäß Anspruch 1, eine Formulierung gemäß Anspruch 14 und eine Dispersion gemäß Anspruch 15. Vorteilhafte Weiterbildungen sind in den Unteransprüchen angegeben. Sie können beliebig kombiniert werden, sofern sich aus dem Kontext nicht eindeutig das Gegenteil ergibt.

Ein Verfahren zur Herstellung einer pharmazeutischen Formulierung umfasst die Schritte:
A) Suspendieren eines pharmazeutischen Wirkstoffes in einer wässrigen Lösung eines Polymers;
B) Trocknen der nach Schritt A) erhaltenen Mischung;
wobei in Schritt A) der pharmazeutische Wirkstoff in Form von Partikeln vorliegt, deren d₉₀-Wert der Partikelgrößenverteilung (volumenbasiert; bestimmt mittels Laserbeugung gemäß ISO 13320:2009) ≤ 1 µm beträgt und vor Schritt B) der pharmazeutische Wirkstoff weiterhin mit einem ionischen Tensid kontaktiert wird.

Die vorliegende Erfindung ermöglicht die prozessunabhängige (bezogen auf die Herkunft der Wirkstoffpartikel) Herstellung redispergierbarer Wirkstoff-Nanopartikel enthaltender Pulver. Dabei handelt es sich um eine Zusammensetzung, die sowohl Wirkstoff in Form von Nano-Partikeln als auch Polymere und ionische Tenside enthält. Dabei kann auf zusätzliche Gerüstbildner vollständig verzichtet werden. Die resultierende PGV der durch erneute Benetzung der Pulver mit Wasser entstandenen Suspension entspricht dabei nahezu der PGV der Ursprungssuspension.

Somit liegt in der nach Schritt B) erhaltenen getrockneten Mischung und auch in einer eventuellen resuspendierten Formulierung der Wirkstoff weiterhin in Form von Partikeln vor, deren d₉₀-Wert der Partikelgrößenverteilung ≤ 1 µm beträgt.

In Schritt A) wird der pharmazeutische Wirkstoff mit einer wässrigen Lösung eines Polymers kontaktiert. Man erhält somit eine Suspension des Wirkstoffes. Mit eingeschlossen in den Begriff der wässrigen Lösung des Polymers sind auch wässrige Gele des Polymers. Dementsprechend ist das Polymer ein wasserlösliches Polymer. Unter "wasserlöslich" ist zu verstehen, dass sich bei 20 °C in 100 g Wasser mindestens 0.5 g, bevorzugt mindestens 2 g des Polymers auflösen beziehungsweise unter Gelbildung lösen.

Das Polymer kann ein neutrales Polymer oder ein kationischer oder anionischer Polyelektrolyt sein und kann ausgewählt sein aus der Gruppe: Alkylcellulosen, Hydroxyalkylcellulosen, Hydroxyalkylalkylcellulosen, Carboxyalkylcellulosen, Alkalimetallsalze der Carboxyalkylcellulosen, Carboxyalkylalkylcellulosen, Carboxyalkylcelluloseester, Stärken, Pektine, Chitinderivate, Polysaccharide, Polyacrylsäure und ihre Salze, Polymethacrylsäure und ihre Salze, Polyvinylalkohol, Polyvinylpyrrolidon, Polyalkylenoxide, Copolymere aus den genannten Polymertypen oder eine Mischung aus mindestens zwei der vorgenannten Polymere.

Beispiele für geeignete Wirkstoffklassen sind Benzodiazepine, Antihypertensiva, Vitamine, Cytostatika - insbesondere Taxol, Anästhetika, Neuroleptika, Antidepressiva, antiviral wirksame Mittel wie beispielsweise Anti-HIV wirksame Mittel, Antibiotika, Antimykotika, Antidementiva, Fungizide, Chemotherapeutika, Urologika, Thrombozytenaggregationshemmer, Sulfonamide, Spasmolytika, Hormone, Immunglobuline, Sera, Schilddrüsentherapeutika, Psychopharmaka, Parkinsonmittel und andere Antihyperkinetika, Ophthalmika, Neuropathiepräparate, Calciumstoffwechselregulatoren, Muskelrelaxantia, Narkosemittel, Lipidsenker, Lebertherapeutika, Koronarmittel, Kardiaka, Immuntherapeutika, regulatorische Peptide und ihre Hemmstoffe, Hypnotika, Sedativa, Gynäkologika, Gichtmittel, Fibrinolytika, Enzympräparate und Transportproteine, Enzyminhibitoren, Emetika, Durchblutungsfördernde Mittel, Diuretika, Diagnostika, Corticoide, Cholinergika, Gallenwegstherapeutika, Antiasthmatika, Broncholytika, Betarezeptorenblocker, Calciumantagonisten, ACE-Hemmer, Arteriosklerosemittel, Antiphlogistika, Antikoagulantia, Antihypotonika, Antihypoglykämika, Antihypertonika, Antifibrinolytika, Antiepileptika, Antiemetika, Antidota, Antidiabetika, Antiarrhythmika, Antianämika, Antiallergika, Anthelmintika, Analgetika, Analeptika, Aldosteronantagonisten, Abmagerungsmittel oder Mischungen aus mindestens zwei der vorgenannten Wirkstoffklassen.Bei entsprechend geringem Zeitabstand zwischen Suspendieren und Trocknen kann auch ein wasserlöslicher Wirkstoff eingesetzt werden, welcher sich in der zur Verfügung stehenden Zeit nicht auflöst. Vorzugsweise ist der Wirkstoff jedoch wasserunlöslich, das heißt dass sich bei 20 °C in 100 g Wasser weniger als 2 g und besonders bevorzugt weniger als 0.5 g des Wirkstoffs lösen.

Hinsichtlich der Partikelgröße des Wirkstoffes ist bevorzugt, dass der d₉₀-Wert der Partikelgrößenverteilung (d₉₀ bedeutet, dass 90% aller Partikel maximal diesen Durchmesser aufweisen; die Bestimmung erfolgt mittels Laserbeugung gemäß ISO 13320:2009) ≥ 10 nm bis ≤ 1 µm beträgt, bevorzugt ≥ 50 nm bis ≤ 500 nm und besonders bevorzugt ≥ 30 nm bis ≤ 300 nm.

Das ionische Tensid kann ein anionisches, kationisches oder zwitterionisches (amphoteres) Tensid sein. Ohne auf eine Theorie festgelegt zu sein wird angenommen, dass das ionische Tensid sich in Kombination mit dem Polymer positiv auf die Stabilität der Wirkstoffpartikel während der Trocknung auswirkt. Die Partikel sind folglich durch die Kombination aus elektrostatischer und sterischer Stabilisierung nahezu vollständig redispergierbar. Zudem kann beobachtet werden, dass ein polymorpher Zustand der Partikel erhalten bleibt. Dieses lässt sich sowohl mittels Röntgenpulverdiffraktometrie als auch mit Fourier-transformierter Infrarot-Spektroskopie dokumentieren.

Die Dosierung der einzelnen Komponenten in Schritt A) kann beispielsweise so erfolgen, dass der Polymergehalt ≥ 0.1 bis ≤ 40 Gewichts-% und der Tensidgehalt ≥ 0.001 bis ≤ 10 Gewichts-%, jeweils bezogen auf das Gesamtgewicht der Suspension in Schritt A), betragen. Ein weiteres Beispiel für eine Dosierung ist ein Gewichtsverhältnis von Wirkstoff: Polymer : Tensid von ≥ 0.01 bis ≤ 5 : 1 : ≥ 0.001 bis ≤ 1.

Die Trocknung in Schritt B) kann zum Beispiel durch Gefriertrocknung, Sprühtrocknung in einem Rotationsverdampfer oder allgemein in einem Kontakttrocknungsverfahren erfolgen. Nach der Trocknung und Redispergierung in solchen wässrigen Medien, die den Wirkstoff nicht vollständig molekular auflösen, weicht die Partikelgrößenverteilung (PGV) der Ursprungssuspension im Vergleich zur PGV des erneut dispergierten Pulvers vorzugsweise nur um den Faktor X in einem Bereich von ≥ 1 bis ≤ 3, bezogen auf den d₉₀-Wert der jeweiligen Suspensionen, ab. Dabei entspricht X dem Quotienten n/v, wobei n für den d₉₀-Wert der PGV nach der Trocknung und erneuten Dispergierung und v für den d₉₀-Wert vor der Trocknung steht. Besonders bevorzugt ist X ≥ 1 bis ≤ 1.2.

In einer weiteren Ausführungsform ist der pharmazeutische Wirkstoff ausgewählt aus: Cyclosporin A, Cyclosprin G, Rapamycin, Tacrolimus, Deoxyspergualin, Mycophenolate-Mofetil, Gusperimus; Acetylsalicylsäure, Ibuprofen, S(+)-Ibuprofen, Indomethacin, Diclofenac, Piroxicam, Meloxicam, Tenoxicam, Naproxen, Ketoprofen, Flurbiprofen, Fenoprofen, Felbinac, Sulindac, Etodolac, Oxyphenbutazon, Phenylbutazon, Nabumeton; Nifedipin, Nitrendipin, Nimodipin, Nisoldipin, Isradipin, Felodipin, Amlodipin, Nilvadipin, Lacidipin, Benidipin, Masnidipin, Furnidipin, Niguldipin; α-Liponsäure; Muramyldipeptid oder -tripeptid, Romurtid; Vitamin A, D, E oder F; Vincopectin, Vincristin, Vinblastin, Reserpin, Codein; Bromocriptin, Dihydroergotamin, Dihydroergocristin; Chlorambucil, Etoposid, Teniposid, Idoxifen, Tallimustin, Teloxantron, Tirapazamin, Carzelesin, Dexniguldipin, Intoplicin, Idarubicin, Miltefosin, Trofosfamid, Teloxantrone, Melphalan, Lomustin, 4,5-Bis(4'fluoranilino)-phthalimid; 4,5-Dianilinophthalimid.; Thymoctonan, Prezatid-Kupferacetat; Erythromycin, Daunorubicin, Gramicidin, Doxorubicin, Amphotericin B, Gentamycin, Leucomycin, Streptomycin, Ganefromycin, Rifamexil, Ramoplanin, Spiramycin; Fluconazol, Ketoconazol, Itraconazol; Famotidin, Cimetidin, Ranitidin, Roxatidin, Nizatidin, Omeprazol; N-[4-Methyl-3-(4-pyridin-3- -ylpyrimidin-2-ylamino)-phenyl]-benzamid, N-Benzyol-staurosporin; BOC-PhecPhe-Val-Phe-Morpholin oder sein O-[2-(2-methoxyethoxy)-acetoxy]-Derivat; N-[4-(5-Cyclopentyloxy-carbonylamino-1-methylindol-3-ylmethyl)-3-methoxybenzoyl]-2-vinyloxy]- benzolsulfonamid oder einer Mischung aus mindestens zwei der vorgenannten Wirkstoffe.

In einer weiteren Ausführungsform ist das Polymer ausgewählt aus: Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Natriumcarboxymethylcellulose, Carboxymethylethylcellulose, Carboxyalkylcelluloseester, Stärken, Natriumcarboxymethylamylopektin, Chitosan, Alginsäure, Alkalimetall- und Ammoniumsalze der Alginsäure, Carrageenane, Galaktomannane, Traganth, Agar-Agar, Gummi arabicum, Guargummi, Xanthangummi, Polyacrylsäure und ihre Salze, Polymethacrylsäure und ihre Salze, Polyvinylalkohol, Polyvinylpyrrolidon, Polyethylenoxid, Polypropylenoxid, Copolymere aus Ethylenoxid und Propylenoxid, N-Vinylpyrrolidon-Vinylacetat-Copolymere oder eine Mischung aus mindestens zwei der vorgenannten Polymere. Besonders bevorzugt sind Polyvinylpyrrolidone (insbesondere K12 und K30-Typen) und N-Vinylpyrrolidon-Vinylacetat-Copolymere.

In einer weiteren Ausführungsform ist das ionische Tensid ausgewählt aus:
Acylaminosäuren (und deren Salze), wie: Acylglutamate, beispielsweise Natriumacylglutamat, Di-TEA-palmitoylaspartat und Natriucaprylglutamat; Acylpeptide, beispielsweise Palmitoylhydrolysiertes Milchprotein, Natrium Cocoylhydrolysiertes Soja Protein und Natrium-/Kalium Cocoyl-hydrolysiertes Kollagen; Sarcosinate, beispielsweise Myristoyl Sarcosin, TEA-lauroyl Sarcosinat, Natriumlauroylsarcosinat und Natriumcocoylsarkosinat; Taurate, beispielsweise Natriumlauroyltaurat und Natriummethylcocoyltaurat; Acyllactylate, Luroyllactylat, Caproyllactylat, Alaninate; Carbonsäuren und Derivate, wie: Carbonsäuren, beispielsweise Laurinsäure, Aluminiumstearat, Magnesiumalkanolat und Zinkundecylenat, Ester-Carbonsäuren, beispielsweise Calciumstearoyllactylat und Natrium PEG-Lauramidcarboxylat, EtherCarbonsäuren, beispielsweise Natriumlaureth-Carboxylat und Natrium PEG-Cocamide Carboxylat; Phosphorsäureester und Salze, wie beispielsweise DEA-Oleth--Phosphat und Dilaureth-Phosphat; Sulfonsäuren und Salze, wie Acyl-isethionate, z.B. Natrium-/ Ammoniumcocoyl-isethionat, Alkylarylsulfonate, Alkylsulfonate, beispielsweise Natriumcocosmonoglyceridsulfat, Natrium C-Olefin-sulfonat, Natriumlaurylsulfoacetat und Magnesium PEG-Cocamidsulfat, Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat; sowie Schwefelsäureester, wie Alkylethersulfat, beispielsweise Natrium-, Ammonium-, Magnesium-, MIPA-, TIPA-Laurethsulfat, Natriummyrethsulfat und Natrium C-Parethsulfat, Alkylsulfate, beispielsweise Natrium-, Ammonium-und TEA-Laurylsulfat.

Erfindungsgemäß können das oder die ionischen Tenside ferner vorteilhaft gewählt werden aus der Gruppe der kationischen Tenside. Vorteilhaft zu verwendende kationische Tenside sind Alkylamine, Alkylimidazole, ethoxylierte Amine, quaternäre Tenside und Esterquats.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder - bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersultate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

Erfindungsgemäß können das oder die ionischen Tenside vorteilhaft gewählt werden aus der Gruppe der amphoteren Tenside.

Vorteilhaft zu verwendende amphotere Tenside sind: Acyl-/dialkylethylendiamine, beispielsweise Natriumacylamphoacetat, Dinatriumacylamphodipropionat, Dinatriumalkylamphodiacetat, Natriumacylamphohydroxypropylsultonat, Dinatriumacylamphodiacetat und Natriumacylamphopropionat sowie N-Alkylaminosäuren, beispielsweise Aminopropylalkylglutamid, Alkylaminopropionsäure, Natuiumalkylimidodipropionat und Lauroamphocarboxyglycinat.

Besonders bevorzugt als Tensid ist Natriumdodecylsulfat (SDS), Natriumdocusat, Natriumoleat und/oder Natriumdesoxycholat.

In einer weiteren Ausführungsform werden die Partikel des Wirkstoffes nicht mit einem Zucker oder Zuckeralkohol kontaktiert. Solche Verbindungen werden im Stand der Technik als Gerüstbildner eingesetzt und sind im erfindungsgemäßen Verfahren entbehrlich.

In einer weiteren Ausführungsform liegen die Partikel des Wirkstoffes wenigstens teilweise in kristalliner Form vor.

In einer weiteren Ausführungsform liegen der Wirkstoff und das Polymer in einem Gewichtsverhältnis von ≥ 1:4 bis ≤ 9:1 (vorzugsweise ≥ 1:1 bis ≤ 2:1) zueinander vor.

In einer weiteren Ausführungsform liegen das Polymer und das Tensid in einem Gewichtsverhältnis von ≥ 10:1 bis ≤ 300:1 (vorzugsweise ≥ 40:1 bis ≤ 100:1) zueinander vor.

In einer weiteren Ausführungsform wurde der Wirkstoff in Form von Partikeln mittels Mahlen erhalten.

In einer weiteren Ausführungsform wurde der Wirkstoff in Form von Partikeln mittels Ausfällung erhalten.

In einer weiteren Ausführungsform erfolgt das Trocknen in Schritt B) mittels Gefriertrocknung.

In einer weiteren Ausführungsform erfolgt das Trocknen in Schritt B) mittels eines auf dem Versprühen der Wirkstoffsuspension basierenden Verfahren. Bevorzugt ist hierbei die Sprühtrocknung.

In einer weiteren Ausführungsform erfolgt das Trocknen in Schritt B) mittels Kontakttrocknung.

In einer weiteren Ausführungsform wird die nach Schritt B) erhaltene getrocknete Mischung anschließend in einem Suspensionsmedium suspendiert. Der Gehalt an Wirkstoff kann ≥ 50 Gewichts-%, vorzugsweise ≥ 60 Gewichts-%, bezogen auf das Gesamtgewicht der Suspension, betragen.

In einer weiteren Ausführungsform ist das Suspensionsmedium ein wässriges Suspensionsmedium. Vorzugsweise wird Wasser ohne weitere Zusätze verwendet.

Die Erfindung betrifft weiterhin eine pharmazeutische Formulierung, umfassend einen mit einem zumindest teilweise in Wasser löslichen Polymer beschichteten pharmazeutischen Wirkstoff, wobei der pharmazeutische Wirkstoff in Form von Partikeln vorliegt, deren d₉₀-Wert der Partikelgrößenverteilung ≤ 1 µm beträgt das Polymer weiterhin ein ionisches Tensid enthält. Diese Formulierung kann durch ein erfindungsgemäßes Verfahren erhalten werden. Entsprechend sind die Ausführungsformen zum Verfahren, die vorstehend erläutert wurden, auch auf die Formulierung übertragbar.

Ein weiterer Aspekt der Erfindung ist eine Suspension eines pharmazeutischen Wirkstoffes, welche durch ein erfindungsgemäßes Verfahren erhältlich ist.

### Beispiele

Die vorliegende Erfindung wird anhand der nachfolgenden Beispiele und Figuren näher erläutert, ohne jedoch darauf beschränkt zu sein. Die Abkürzung "wt%" bedeutet Gewichtsprozent und sind auf das Gesamtgewicht der wässrigen Suspension bezogen. PVP K12 ist ein Polyvinylpyrrolidon mit einem K-Wert (nach Fikentscher, DIN EN ISO 1628-1)von 12. SDS ist Natriumdodecylsulfat. KVA 64 ist Kollidon® VA64, ein Vinylpyrrolidon-Vinylacetat-Copolymer.

### Beispiel 1: Gefriertrocknung von Indomethacin-PVP K12-SDS-Nanosuspensionen

Die Nanosuspension wurde mittels Planetenkugelmühle (Fritsch Pulverisette 5) hergestellt. Dazu wurden 10 wt% Indomethacin mit 6 wt% PVP K12 und 0,1 wt% SDS stabilisiert. Die Polymer-Tensid-Lösungen wurden separat hergestellt und gelöst. Anschließend wurde die Lösung mit Indomethacin-Pulver versetzt und die entstandene Suspension auf einer Rührplatte homogenisiert. Die Mahlräume wurden zu 60% (Volumen) mit 0,4-0,6 mm (SiLibeads, Zirkonoxid, Yttrium stabilisiert) Mahlkörpern und das Restvolumen mit Suspension, blasenfrei befüllt. Nach 1 h 30 min Mahldauer bei 400 rpm lag eine Nanosuspension mit Partikeln d₉₀ < 500 nm (Malvern, Mastersizer 2000) vor, die zur Trocknung verwendet werden konnte.

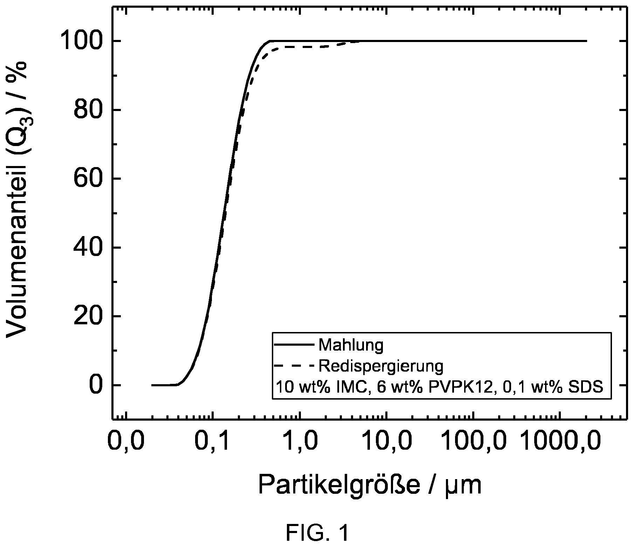

Zur Gefriertrocknung wurden 3 ml Vials mit 0,7 g Suspension (Füllstand < 1 cm) befüllt und in den auf -40°C vorgekühlten Gefriertrockner gestellt. Nach der Trocknung wurden die resultierenden "Feststoffkuchen" mit einem Spatel zu Pulver zerdrückt und mit Wasser benetzt. Die entstandene Suspension wurde dann mittels statischer Lichtstreuung (Malvern, Mastersizer 2000) vermessen und mit der Partikelgrößenverteilung der Ursprungssuspension nach der Mahlung verglichen (Abb. 1). Dabei zeigten die Suspensionen, die 0,1 wt% SDS und 6 wt% PVPK12 enthielten, nahezu vollständige Redispergierbarkeit (10 wt% Indomethacin). Der resultierende Wirkstoffgehalt der redispergierbaren, Wirkstoff- Nanopartikelenthaltenden Pulver betrugt über 60 wt%.

FIG. 1 zeigt die Partikelgrößenverteilung vor der Trocknung (nach der Mahlung) und nach der Redispergierung (X=1.120).

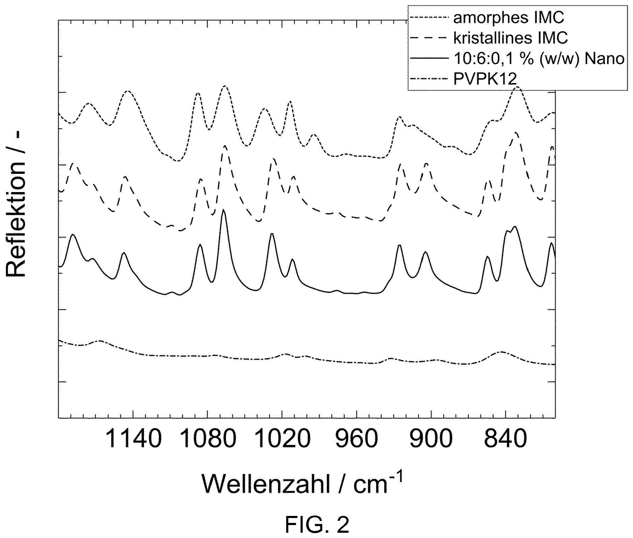

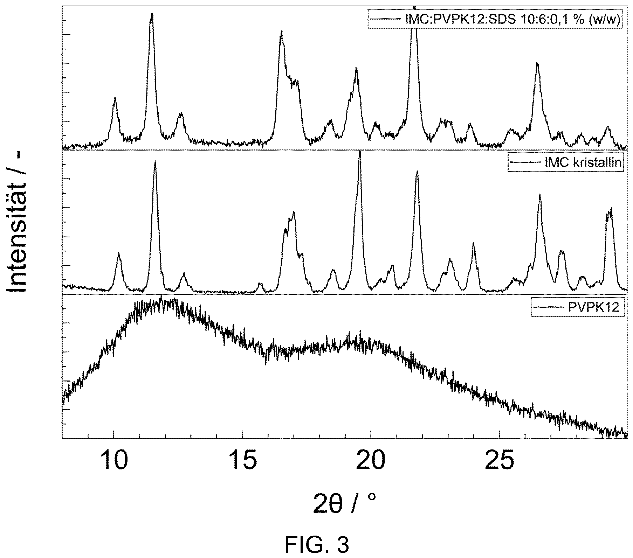

Die Nanopartikel enthaltenden Pulver wurden zusätzlich mittels Fouriertransform-Infrarotspektroskopie (FTIR, FIG. 2) und Röntgenpulverdiffraktometrie (XRPD, FIG. 3) untersucht. Man erkennt, dass der kristalline Zustand der Indomethacin-Partikel erhalten blieb.

### Beispiel 2: Gefriertrocknung von Indomethacin-KVA 64-SDS-Nanosuspensionen

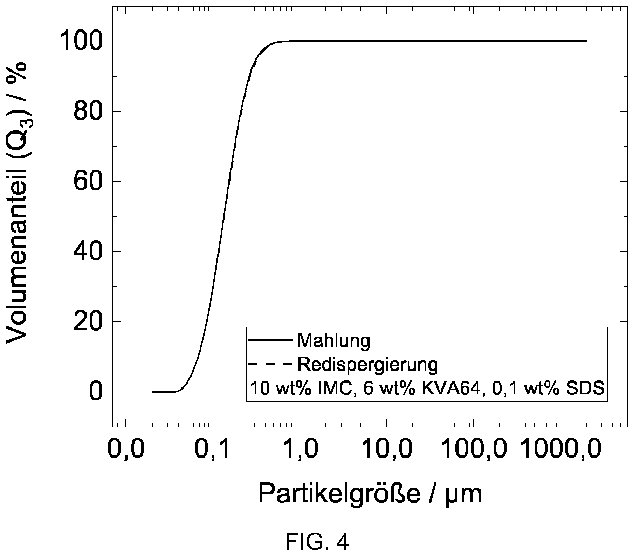

Die Nanosuspension wurde analog zu Beispiel 1 hergestellt, mit dem Unterschied, dass anstatt PVP K12 das Polymer KVA 64 verwendet wurde (10:6:0,1 wt% Wirkstoff:Polymer:SDS). Zur Gefriertrocknung wurden 3 ml Vials mit 0,7 g Suspension (Füllstand < 1 cm) befüllt und in den auf -40°C vorgekühlten Gefriertrockner gestellt. Nach der Trocknung wurden die resultierenden "Feststoffkuchen" mit einem Spatel zu Pulver zerdrückt und mit Wasser benetzt. Die entstandene Suspension wurde dann mittels statischer Lichtstreuung (Malvern, Mastersizer 2000) vermessen und mit der Partikelgrößenverteilung der Ursprungssuspension nach der Mahlung verglichen (FIG. 4). Dabei zeigten die Suspensionen die 0,1 wt% SDS und 6 wt% KVA 64 enthielten nahezu vollständige Redispergierbarkeit (10wt% Indomethacin). Der resultierende Wirkstoffgehalt der redispergierbaren, Wirkstoff- Nanopartikelenthaltenden Pulver betrugt über 60 wt%.

FIG. 4 zeigt die Partikelgrößenverteilung vor der Trocknung (nach der Mahlung) und nach der Redispergierung (X=1.022).

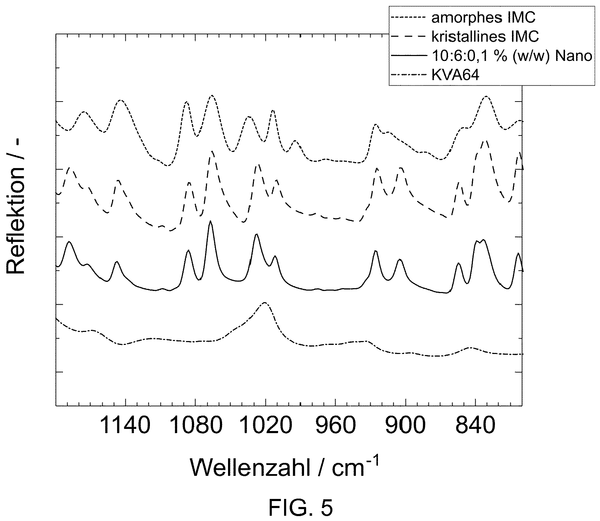

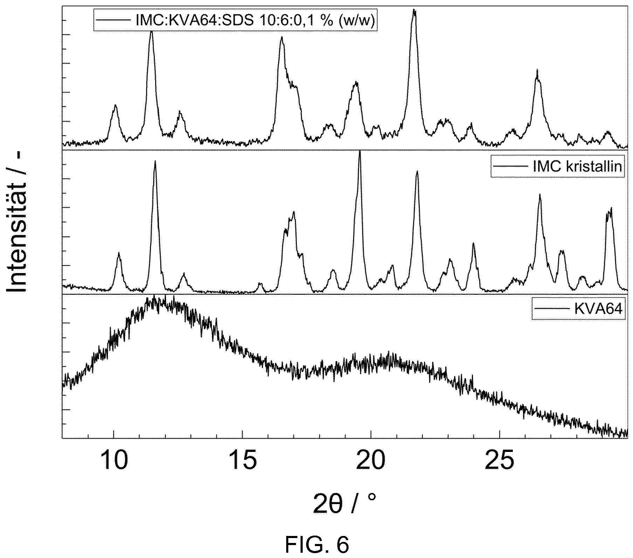

Die Nanopartikel enthaltenden Pulver wurden zusätzlich mittels Fouriertransform-Infrarotspektroskopie (FTIR, FIG. 5) und Röntgenpulverdiffraktometrie (XRPD, FIG. 6) untersucht. Man erkennt, dass der kristalline Zustand der Indomethacin-Partikel erhalten blieb.

### Beispiel 3: Gefriertrocknung von Verciguat-PVP K12-SDS-Nanosuspensionen

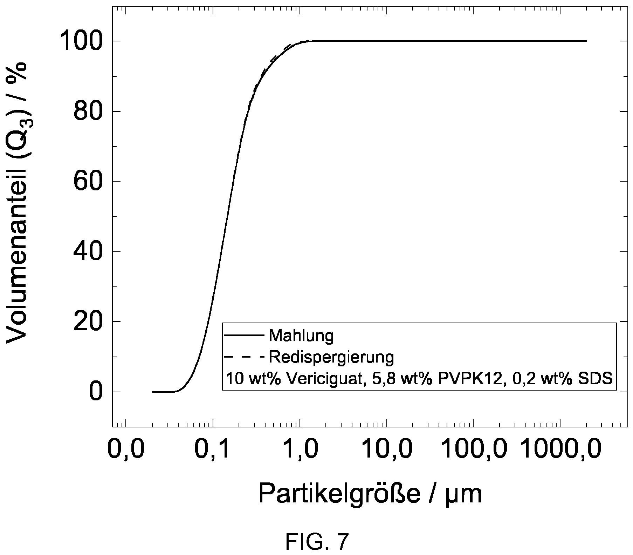

Die Nanosuspension wurde analog zu Beispiel 1 hergestellt, mit dem Unterschied, dass anstatt Indomethacin, Vericiguat verwendet wurde und andere Konzentrationsverhältnisse vorlagen (10:5,8:0,2 wt% Wirkstoff:Polymer:SDS). Zur Gefriertrocknung wurden 3 ml Vials mit 0,7 g Suspension (Füllstand < 1 cm) befüllt und in den auf -40°C vorgekühlten Gefriertrockner gestellt. Nach der Trocknung wurden die resultierenden "Feststoffkuchen" mit einem Spatel zu Pulver zerdrückt und mit Wasser benetzt. Die entstandene Suspension wurde dann mittels statischer Lichtstreuung (Malvern, Mastersizer 2000) vermessen und mit der Partikelgrößenverteilung der Ursprungssuspension nach der Mahlung verglichen (FIG. 7). Dabei zeigten die Suspensionen die 0,2 wt% SDS und 5,8 wt% PVPK12 enthielten nahezu vollständige Redispergierbarkeit (10wt% Vericiguat). Der resultierende Wirkstoffgehalt der redispergierbaren, Wirkstoff-Nanopartikelenthaltenden Pulvern betrugt über 60 wt%.

FIG. 7 zeigt die Partikelgrößenverteilung vor der Trocknung (nach der Mahlung) und nach der Redispergierung.

### Vergleichsbeispiel: Gefriertrocknung von Indomethacin-PVP K12-Nanosuspensionen ohne Tensid

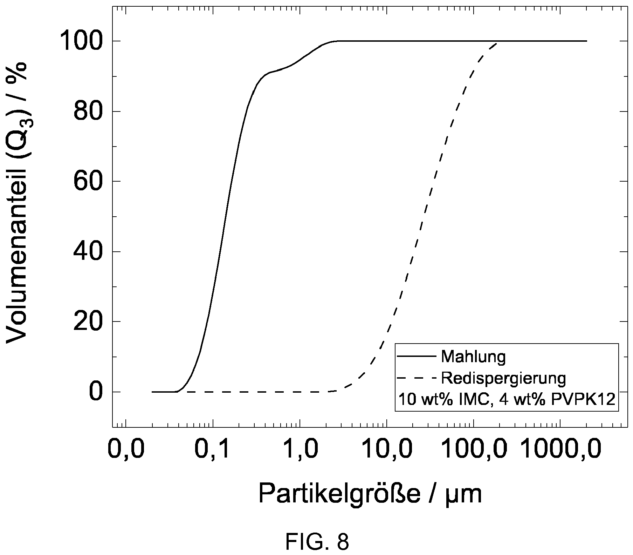

Die Nanosuspension wurde analog zu Beispiel 1 hergestellt, mit dem Unterschied, dass vollständig auf Tensid verzichtet wurde. Dies hatte keinen großen Einfluss auf das Mahlergebnis, sodass auch hier Partikel < 500 nm stabil herstellbar waren. Zur Gefriertrocknung wurden 3 ml Vials mit 0,7 g Suspension (Füllstand < 1 cm) befüllt und in den auf -40°C vorgekühlten Gefriertrockner gestellt. Nach der Trocknung wurden die resultierenden "Feststoffkuchen" mit einem Spatel zu Pulver zerdrückt und mit Wasser benetzt. Die entstandene Suspension wurde dann mittels statischer Lichtstreuung (Malvern, Mastersizer 2000) vermessen und mit der Partikelgrößenverteilung der Ursprungssuspension nach der Mahlung verglichen (FIG. 8). Dabei zeigten die Suspensionen unabhängig vom Polymergehalt keine ausreichende Redispergierbarkeit (10 wt% Wirkstoff). FIG. 8 zeigt die Partikelgrößenverteilung vor der Trocknung (nach der Mahlung) und nach der Redispergierung (X=242.823).

## Patentansprüche

1. Verfahren zur Herstellung einer pharmazeutischen Formulierung, umfassend die Schritte:
A) Suspendieren eines pharmazeutischen Wirkstoffes in einer wässrigen Lösung eines Polymers;
B) Trocknen der nach Schritt A) erhaltenen Mischung;
**dadurch gekennzeichnet, dass**
in Schritt A) der pharmazeutische Wirkstoff in Form von Partikeln vorliegt, deren d₉₀-Wert der Partikelgrößenverteilung ≤ 1 µm beträgt und dass
vor Schritt B) der pharmazeutische Wirkstoff weiterhin mit einem ionischen Tensid kontaktiert wird.

2. Verfahren gemäß Anspruch 1, wobei der pharmazeutische Wirkstoff ausgewählt ist aus: Cyclosporin A, Cyclosprin G, Rapamycin, Tacrolimus, Deoxyspergualin, Mycophenolate-Mofetil, Gusperimus; Acetylsalicylsäure, Ibuprofen, S(+)-Ibuprofen, Indomethacin, Diclofenac, Piroxicam, Meloxicam, Tenoxicam, Naproxen, Ketoprofen, Flurbiprofen, Fenoprofen, Felbinac, Sulindac, Etodolac, Oxyphenbutazon, Phenylbutazon, Nabumeton; Nifedipin, Nitrendipin, Nimodipin, Nisoldipin, Isradipin, Felodipin, Amlodipin, Nilvadipin, Lacidipin, Benidipin, Masnidipin, Furnidipin, Niguldipin; α-Liponsäure; Muramyldipeptid oder -tripeptid, Romurtid; Vitamin A, D, E oder F; Vincopectin, Vincristin, Vinblastin, Reserpin, Codein; Bromocriptin, Dihydroergotamin, Dihydroergocristin; Chlorambucil, Etoposid, Teniposid, Idoxifen, Tallimustin, Teloxantron, Tirapazamin, Carzelesin, Dexniguldipin, Intoplicin, Idarubicin, Miltefosin, Trofosfamid, Teloxantrone, Melphalan, Lomustin, 4,5-Bis(4'fluoranilino)-phthalimid; 4,5-Dianilinophthalimid.; Thymoctonan, Prezatid-Kupferacetat; Erythromycin, Daunorubicin, Gramicidin, Doxorubicin, Amphotericin B, Gentamycin, Leucomycin, Streptomycin, Ganefromycin, Rifamexil, Ramoplanin, Spiramycin; Fluconazol, Ketoconazol, Itraconazol; Famotidin, Cimetidin, Ranitidin, Roxatidin, Nizatidin, Omeprazol; N-[4-Methyl-3-(4-pyridin-3- -ylpyrimidin-2-ylamino)-phenyl]-benzamid, N-Benzyol-staurosporin; BOC-PhecPhe-Val-Phe-Morpholin oder sein O-[2-(2-methoxyethoxy)-acetoxy]-Derivat; N-[4-(5-Cyclopentyloxy-carbonylamino-1-methylindol-3-ylmethyl)-3-methoxybenzoyl]-2-vinyloxy]- benzolsulfonamid oder einer Mischung aus mindestens zwei der vorgenannten Wirkstoffe.

3. Verfahren gemäß Anspruch 1 oder 2, wobei das Polymer ausgewählt ist aus Methylcellulose, Hydroxymethylcellulose, Hydroxyethylcellulose, Hydroxypropylcellulose, Hydroxybutylcellulose, Hydroxyethylmethylcellulose, Hydroxypropylmethylcellulose, Carboxymethylcellulose, Natriumcarboxymethylcellulose, Carboxymethylethylcellulose, Carboxyalkylcelluloseester, Stärken, Natriumcarboxymethylamylopektin, Chitosan, Alginsäure, Alkalimetall- und Ammoniumsalze der Alginsäure, Carrageenane, Galaktomannane, Traganth, Agar-Agar, Gummi arabicum, Guargummi, Xanthangummi, Polyacrylsäure und ihre Salze, Polymethacrylsäure und ihre Salze, Polyvinylalkohol, Polyvinylpyrrolidon, Polyethylenoxid, Polypropylenoxid, Copolymere aus Ethylenoxid und Propylenoxid, N-Vinylpyrrolidon-Vinylacetat-Copolymere oder eine Mischung aus mindestens zwei der vorgenannten Polymere.

4. Verfahren gemäß einem der Ansprüche 1 bis 3, wobei das ionische Tensid ausgewählt ist aus: Acylaminosäuren (und deren Salze), Carbonsäuren und Derivate, Sulfonsäuren und Salze, Schwefelsäureester, Alkylamine, Alkylimidazole, ethoxylierte Amine, quaternäre Tenside, Esterquats, amphotere Tenside oder eine Mischung aus mindestens zwei der vorgenannten Tenside.

5. Verfahren gemäß einem der Ansprüche 1 bis 4, wobei die Partikel des Wirkstoffes nicht mit einem Zucker oder Zuckeralkohol kontaktiert werden.

6. Verfahren gemäß einem der Ansprüche 1 bis 5, wobei der Wirkstoff und das Polymer in einem Gewichtsverhältnis von ≥ 1:4 bis ≤ 9:1 zueinander vorliegen.

7. Verfahren gemäß einem der Ansprüche 1 bis 6, wobei das Polymer und das Tensid in einem Gewichtsverhältnis von ≥ 10:1 bis ≤ 300:1 zeinander vorliegen.

8. Verfahren gemäß einem der Ansprüche 1 bis 7, wobei der Wirkstoff in Form von Partikeln mittels Mahlen erhalten wurde.

9. Verfahren gemäß einem der Ansprüche 1 bis 7, wobei der Wirkstoff in Form von Partikeln mittels Ausfällung erhalten wurde.

10. Verfahren gemäß einem der Ansprüche 1 bis 9, wobei das Trocknen in Schritt B) mittels Gefriertrocknung erfolgt.

11. Verfahren gemäß einem der Ansprüche 1 bis 9, wobei das Trocknen in Schritt B) mittels eines auf dem Versprühen der Wirkstoffsuspension basierendem Verfahrens erfolgt.

12. Verfahren gemäß einem der Ansprüche 1 bis 9, wobei das Trocknen in Schritt B) mittels Kontakttrocknung erfolgt.

13. Verfahren gemäß einem der Ansprüche 1 bis 12, wobei die nach Schritt B) erhaltene getrocknete Mischung anschließend in einem Suspensionsmedium suspendiert wird.

14. Pharmazeutische Formulierung, umfassend einen mit einem zumindest teilweise in Wasser löslichen Polymer beschichteten pharmazeutischen Wirkstoff,
**dadurch gekennzeichnet, dass**
der pharmazeutische Wirkstoff in Form von Partikeln vorliegt, deren d₉₀-Wert der Partikelgrößenverteilung ≤ 1 µm beträgt und dass
das Polymer weiterhin ein ionisches Tensid enthält.

15. Suspension eines pharmazeutischen Wirkstoffes, erhältlich durch ein Verfahren gemäß Anspruch 12 oder 13.
